# FASCICULE DE BREVET EUROPEEN

(11) **EP 0 813 880 B1**
(45) Date de publication et mention de la délivrance du brevet: **02.10.2002**
(21) Numéro de dépôt: 97420087.5
(22) Date de dépôt: 09.06.1997
(51) Int. Cl.: A61M 1/16

(54) **Procédé de désinfection pour une machine de dialyse**
Verfahren zur Desinfektion einer Dialysemaschine
Method for desinfecting a dialysis machine

(30) Priorité: 18.06.1996 FR 9607822
(43) Date de publication de la demande: 29.12.1997
(73) Titulaire: HOSPAL INDUSTRIE, F-69883 Meyzieu Cédex (FR)
(72) Inventeur: Pedrazzi, Renato, 41037 Mirandola Modena (IT); Simard, Laurent, 69230 Saint Genis Laval (FR)
(74) Mandataire: Lejeune, Daniel

(56) Documents cités:
- US-A- 4 399 030

## Description

La présente invention a pour objet un procédé de désinfection pour une machine de dialyse.

Une machine de dialyse a pour fonctions principales la préparation d'un liquide de dialyse, l'alimentation d'un dialyseur en liquide de dialyse et l'évacuation du liquide usé (mélange de liquide de dialyse chargé en déchets du métabolisme et d'eau plasmatique) hors du dialyseur.

Le liquide de dialyse est préparé par un mélange dosé d'eau et de deux solutions concentrées contenant les principaux électrolytes du sang. Le mélange est maintenu à température sensiblement constante par chauffage de l'eau, et, dans certaines machines, il est filtré, de façon que le liquide mis en circulation dans le dialyseur soit exempt de germes et d'éléments pyrogènes. Le liquide de dialyse frais filtré peut être filtré une deuxième fois pour servir de liquide de perfusion.

Une machine de dialyse comprend un circuit hydraulique constitué d'un ensemble de canalisations, de pompes, de vannes, de filtres, etc. qui sont susceptibles d'un encrassement propre à favoriser le développement d'une faune bactérienne indésirable. Il est utile de filtrer le liquide de dialyse car, même si dans un dialyseur le sang est isolé du liquide de dialyse par une membrane semi-perméable qui, en principe, ne laisse pas passer les bactéries, une contamination du sang est toujours possible à l'occasion de microfuites dans la membrane. En outre les dérivés bactériens de faible masse moléculaire peuvent traverser les membranes des dialyseurs.

Les zones de la machine qui sont le plus sujettes à encrassement sont les filtres utilisés pour filtrer le liquide de dialyse, dans la première chambre desquels se concentrent tous les matériaux arrêtés par la membrane de ces filtres, ainsi que tout le circuit d'évacuation du liquide usé qui est au contact de substances organiques (déchets du métabolisme, eau plasmatique).

Les machines de dialyse courantes sont donc munies d'un système de désinfection permettant de remplir le circuit hydraulique avec un liquide de désinfection, puis, de l'en purger, après que le désinfectant y a stagné pendant un temps déterminé. Le circuit hydraulique est alors rincé à l'eau et il reste rempli d'eau jusqu'à la prochaine utilisation de la machine. Un tel procédé est décrit dans le document US-A-4 399 030. En général, les machines sont désinfectées entre deux séances de dialyse. Après la dernière séance de dialyse de la journée, selon le type de désinfectant utilisé, soit les machines restent remplies de liquide de désinfection pour la nuit, soit elles sont rincées à l'eau après désinfection et elles restent remplies d'eau pour la nuit. La différence de traitement vient de ce que certains désinfectants (désinfectants à base de chlore, par exemple) sont corrosifs et que leur stagnation prolongée dans la machine en endommagerait le circuit hydraulique.

Il y a un inconvénient à laisser les machines remplies d'eau pendant plusieurs heures : si la désinfection n'a pas éliminé tous les germes présents dans le circuit hydraulique, les germes survivants vont pouvoir se multiplier et coloniser certaines zones du circuit où l'action de la désinfection est moins efficace.

D'un autre côté, les désinfectants à base de chlore, tel l'eau de Javel, sont réputés être les plus efficaces et ils ont un effet additionnel de nettoyage du circuit (dépôts organiques) dont les autres désinfectants couramment utilisés (désinfectants à base d'aldéhyde et désinfectants à base d'acide peracétique) sont dépourvus.

Un but de l'invention est de proposer un système de désinfection pour machine de dialyse qui permette l'utilisation de désinfectants corrosifs sans que cette utilisation n'ait pour conséquence l'endommagement du circuit hydraulique de la machine, ni sa colonisation possible par des germes durant les périodes d'inactivité prolongée de la machine.

Pour atteindre ce but, on prévoit, selon l'invention, un procédé de désinfection du circuit hydraulique d'une machine de dialyse comprenant une entrée pour raccordement à une source d'eau extérieure et une pluralité de canalisations ouvertes prévues pour être reliées, dans une configuration opérationnelle de la machine, à différents accessoires consommables, comprenant les étapes de :
- fermer les canalisations ouvertes sur le circuit hydraulique de façon à former un réseau clos de canalisations ;
- mettre en communication avec le circuit hydraulique, à proximité de l'entrée, un réservoir contenant un liquide de désinfection concentré ;
- provoquer un écoulement proportionnel d'eau et de liquide de désinfection concentré dans le circuit hydraulique de façon à produire un premier liquide ayant un pouvoir bactéricide ;
caractérisé en ce qu'il comprend les étapes de :
- interrompre l'écoulement d'eau et de liquide de désinfection concentré lorsque le circuit hydraulique est rempli de liquide bactéricide, le volume initial de liquide de désinfection concentré dans le réservoir étant choisi pour qu'à la fin du remplissage du circuit hydraulique il reste dans le réservoir un volume résiduel ;
- ajouter un volume d'eau au volume résiduel dans le réservoir pour produire un deuxième liquide ;
- vidanger le circuit hydraulique du liquide bactéricide ;
- provoquer simultanément un écoulement proportionnel d'eau et du deuxième liquide dans le circuit hydraulique de façon à produire un troisième liquide ayant un pouvoir bactériostatique et pouvant rester dans le circuit hydraulique pendant au moins douze heures sans y causer de corrosion ; et
- remplir le circuit hydraulique de liquide bactériostatique.

Grâce à ce procédé, il est possible d'utiliser un désinfectant corrosif pour désinfecter une machine de dialyse sans devoir laisser le circuit hydraulique rempli d'eau entre deux séances de traitement séparées par plusieurs heures. La stagnation d'un liquide bactériostatique prévient la formation d'un biofilm dans le circuit hydraulique. Un autre avantage de ce procédé est qu'il est simple à mettre en oeuvre et qu'il offre une sécurité absolue puisqu'il repose sur l'utilisation d'un liquide bactéricide et d'un liquide bactériostatique de même nature chimique dont un mélange accidentel ne causerait naturellement aucune réaction indésirable (comme cela pourrait être le cas, si ces deux liquides étaient de natures chimiques différentes).

Selon une caractéristique de l'invention, préalablement à l'étape de mettre en communication le réservoir avec le circuit hydraulique, le procédé comprend l'étape de remplir le réservoir avec un volume déterminé de liquide de désinfection concentré.

Selon une autre caractéristique de l'invention, préalablement à l'étape de mettre en communication le réservoir avec le circuit hydraulique, le procédé comprend l'étape de remplir le réservoir avec un volume déterminé d'eau et d'y introduire une quantité déterminée de désinfectant soluble sous forme de poudre, de granulés ou de pastilles.

Selon encore une autre caractéristique de l'invention, l'étape de remplissage du circuit hydraulique avec le liquide bactéricide et l'étape de remplissage du circuit hydraulique avec le liquide bactériostatique sont effectuées en plusieurs phases correspondant au remplissage de plusieurs portions du circuit hydraulique.

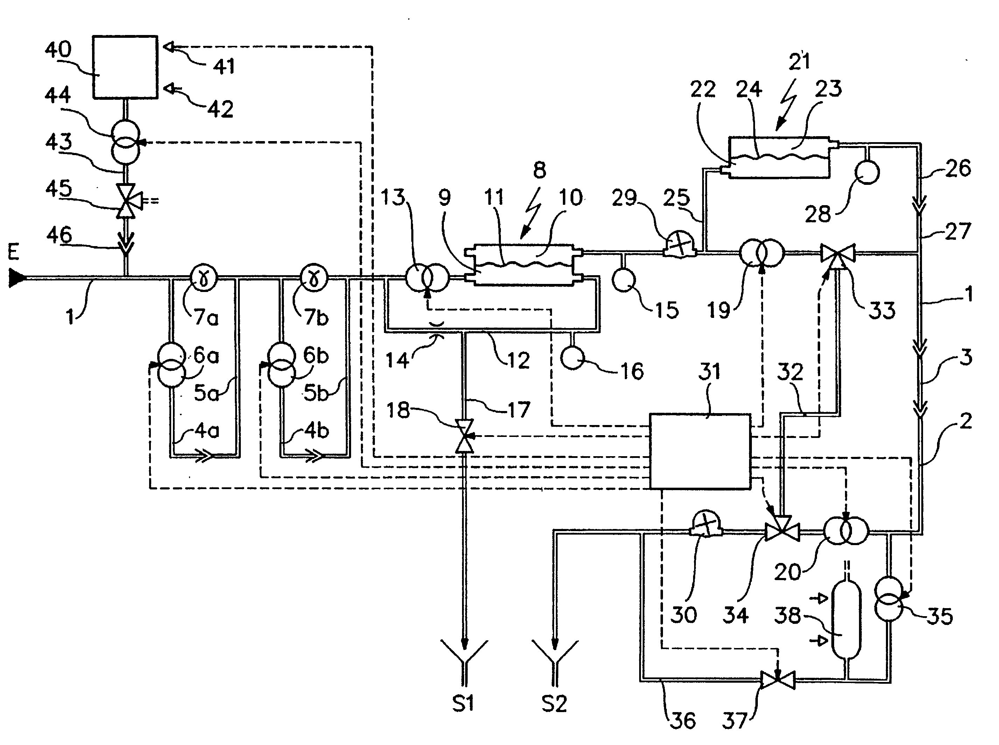

D'autres caractéristiques et avantages de l'invention apparaîtront à la lecture de la description qui va suivre. On se reportera au dessin annexé dont l'unique figure représente le schéma simplifié d'une machine de dialyse dont le circuit hydraulique est disposé dans une configuration non opérationnelle (configuration de désinfection).

A des fins de clarté, ont été omis sur la figure un certain nombre d'organes et d'accessoires dont sont munies les machines de dialyse classiques, et dont la représentation et la description n'aideraient pas à la compréhension de l'invention (tels que, par exemple, un régulateur de pression d'arrivée d'eau, un dispositif de chauffage d'eau, des dispositifs de dégazage d'eau et du liquide de dialyse, un échangeur thermique, etc.).

La machine de dialyse représentée sur la figure comprend une unité de commande et un circuit hydraulique qui est agencé dans une configuration non opérationnelle (par opposition une configuration opérationnelle permettant l'administration d'un traitement).

Le circuit hydraulique comprend une canalisation 1 d'alimentation en liquide de dialyse frais destinée à relier un générateur de liquide de dialyse à une entrée d'un hémodialyseur, et une canalisation 2 d'évacuation de liquide usé destinée à relier une sortie d'un hémodialyseur à l'égout. Comme la machine est en configuration non opérationnelle, la canalisation d'alimentation 1 et la canalisation d'évacuation 2 sont raccordées par une canalisation de jonction 3 dont les extrémités sont munies de d'éléments de raccord identiques à ceux d'un hémodialyseur.

Le générateur de liquide de dialyse comprend un premier dispositif pour le mélange d'une première solution concentrée avec de l'eau provenant d'une source extérieure à la machine (branchement prévu à l'entrée E, à l'extrémité amont de la canalisation d'alimentation 1), et un second dispositif pour le mélange d'une seconde solution concentrée avec la solution diluée produite par le premier dispositif de mélange. Chaque dispositif de mélange comprend une canalisation d'injection 4a, 4b ayant une extrémité connectée à la canalisation d'alimentation 1 et une extrémité munie d'un raccord pour connecter la canalisation d'injection 4a, 4b soit à une source de solution concentrée (configuration opérationnelle) soit à une canalisation de bouclage 5a, 5b (configuration non opérationnelle). La canalisation d'injection 4a, 4b est munie d'une pompe doseuse 6a, 6b dont le débit est réglé en fonction d'une information fournie par une sonde de conductivité 7a, 7b disposée sur la canalisation d'alimentation 1, en aval de la jonction de la canalisation d'injection 4a, 4b à la canalisation d'alimentation 1.

Le circuit hydraulique comprend en outre un premier filtre 8 pour filtrer le liquide de dialyse produit par le générateur de liquide de dialyse. Le filtre 8 a une première et une seconde chambres 9, 10 séparées par une membrane de filtration 11, la première chambre 9 ayant une entrée connectée à une première portion de la canalisation d'alimentation 1 et la seconde chambre 10 ayant une sortie connectée à une seconde portion de la canalisation d'alimentation 1. Une canalisation de recirculation 12 relie une sortie de la première chambre 9 du filtre 8 à l'entrée de cette première chambre. Sur cette canalisation de recirculation 12 sont disposés une pompe de balayage 13, immédiatement en amont de la première chambre 9 du filtre 8, et un organe de rétreint 14, éventuellement réglable.

Deux capteurs de pression 15, 16 sont disposés respectivement sur la canalisation d'alimentation 1 et sur la canalisation de recirculation 12, en sortie de la première et de la deuxième chambres 9, 10 du filtre 8.

Une canalisation de purge 17 de la première chambre 9 du filtre 8, sur laquelle est disposée une vanne 18, est connectée à la canalisation de recirculation 12 entre la sortie de la première chambre 9 du filtre 8 et l'organe de rétreint 14. La canalisation de purge 17 est reliée à l'égout par une première sortie S1 de la machine de dialyse distincte d'une seconde sortie S2 qui forme l'extrémité de la canalisation d'évacuation 2 du liquide usé.

Le liquide de dialyse est mis en circulation dans le circuit hydraulique par une première pompe 19 disposée sur la canalisation d'alimentation 1, en aval du filtre 8, et par une deuxième pompe 20 disposée sur la canalisation d'évacuation 2.

Le circuit hydraulique comporte en outre un générateur de liquide de perfusion comprenant un second filtre 21 ayant une première et une seconde chambre 22, 23 séparées par une membrane de filtration 24. La première chambre 22 a une entrée reliée par une canalisation 25 à la canalisation d'alimentation 1, entre le premier filtre 8 et la première pompe de circulation 19 de liquide de dialyse. La seconde chambre 23 a une sortie reliée à une canalisation de perfusion 26 dont l'extrémité libre est munie d'un connecteur pour raccorder la canalisation de perfusion 26 soit à une canalisation de bouclage 27 connectée à la canalisation d'alimentation 1 (configuration non opérationnelle), soit à une canalisation connectée à un piège à bulles d'un circuit pour la circulation extracorporelle du sang (configuration opérationnelle). Un capteur de pression 28 est disposé sur la canalisation de perfusion 26.

Le circuit hydraulique comprend aussi un système de maîtrise volumétrique de l'ultrafiltration comprenant un premier débitmètre 29, disposé sur la canalisation d'alimentation 1 entre le premier filtre 8 et la première pompe de circulation 19, et un second débitmètre 30, disposé sur la canalisation d'évacuation 2 en aval de la deuxième pompe de circulation 20. Les mesures effectuées par les deux débitmètres sont comparées par l'unité de calcul et de commande 31 qui pilote la deuxième pompe de circulation 20 de façon que les débits mesurés par les deux débitmètres soient identiques. Une canalisation d'étalonnage 32 relie la canalisation d'alimentation 1 à la canalisation d'évacuation 2, par l'intermédiaire de deux vannes trois voies 33, 34 disposées pour l'une en aval de la première pompe de circulation 19 et, pour l'autre, entre la seconde pompe de circulation 20 et le second débitmètre 30. Une pompe d'ultrafiltration 35 est disposée sur une canalisation 36 de dérivation à la canalisation d'évacuation 2, l'extrémité amont de la canalisation de dérivation 36 étant connectée à la canalisation d'évacuation 2 en amont de la seconde pompe 20 et l'extrémité aval de la canalisation de dérivation 36 étant connectée à la canalisation d'évacuation 2 en aval du second débitmètre 30. Un dispositif de mesure du débit de la pompe d'ultrafiltration 35, comprenant une vanne 37 et une burette 38 munie de deux détecteurs de niveau, est disposé sur la canalisation de dérivation 36 en aval de la pompe d'ultrafiltration 35. Ce dispositif de mesure de débit est décrit de façon détaillée dans le document EP 0 403 401.

Le circuit hydraulique comporte enfin un dispositif de désinfection comprenant un réservoir 40 muni d'un détecteur de niveau haut 41 et d'un détecteur de niveau bas 42. Une canalisation 43 relie la base du réservoir 40 à la canalisation d'alimentation 1 en amont du générateur de liquide de dialyse, à proximité de l'orifice d'entrée E de la machine. Cette canalisation est munie (dans l'ordre à partir du réservoir 40) d'une pompe 44, d'une vanne trois voies 45 permettant de mettre en communication, par l'intermédiaire d'une canalisation non représentée, le réservoir 40 et la burette 38, et un connecteur 46 permettant de relier le réservoir 40 à une source de liquide, en particulier à une source d'eau ou de liquide de désinfection concentré.

L'unité de calcul et de commande 31 est reliée à tous les instruments de mesure du circuit hydraulique qui vient d'être décrit (sondes de conductivité, capteurs de pression, débitmètres, détecteurs de niveau, etc.). Par ailleurs, elle est reliée à toutes les pompes et les vannes dont elle commande les débits, l'ouverture et la fermeture en fonction de valeurs de consignes, des mesures effectuées par les instruments de mesure, et de programmes décrivant tous les cycles de fonctionnement de la machine, en particulier le cycle de désinfection selon l'invention.

Conformément à l'invention, la préparation de la machine en vue d'un état non opérationnel prolongé est effectuée de la manière suivante. A la fin d'une séance de traitement, qui se déroule conformément à la description qui en est donnée dans le document EP 0 694 312, toutes les canalisations ouvertes du circuit hydraulique, c'est-à-dire les canalisations ayant une extrémité connectée à un accessoire consommable, sont bouclées sur le circuit hydraulique au moyen des canalisations de bouclage ou de jonction prévues à cet effet (canalisations 4a, 4b d'injection de solutions concentrées utilisées pour la préparation du liquide de dialyse ; canalisation de perfusion 26 ; canalisations d'alimentation 1 et d'évacuation 2). Les canalisations 4a, 4b, 26 sont ainsi bouclées sur le circuit hydraulique au moyen des canalisations de bouclage 5a, 5b, 27, et les canalisations 1 et 2 raccordées par la canalisation de jonction 3.

Le circuit hydraulique forme alors un réseau clos de canalisations ayant une entrée E pour l'eau et les deux sorties S1, S2 décrites plus haut. Il est rempli pour une part de liquide de dialyse frais et, pour une part de liquide usé.

Le raccord 46 placé sur la canalisation 43 d'injection de liquide de désinfection est ouvert et le tronçon de canalisation solidaire du réservoir 40 est relié à une source de liquide de désinfection concentré. Le réservoir 40 est rempli de liquide de désinfection jusqu'au niveau haut 41 au moyen de la pompe 44, puis la canalisation d'injection 43 est connectée à nouveau à la canalisation d'alimentation 1.

La procédure de remplissage du circuit hydraulique avec un liquide de désinfection est alors démarrée par l'unité de commande 31 selon un programme préétabli comprenant plusieurs phases correspondant à la vidange et au remplissage simultanés de différentes portions du circuit. Pendant toute cette procédure, la pompe de balayage 13 et la pompe 44 de liquide de désinfection fonctionnent en permanence. Le débit de cette dernière est ajusté de façon que le mélange (premier liquide ou liquide bactériostatique) d'eau et de liquide de désinfection concentré qui se forme à la jonction de la canalisation d'alimentation 1 et de la canalisation d'injection 43 soit bactéricide. A titre d'exemple, si le liquide de désinfection concentré choisi est de l'eau de Javel à 48° chlorométrique, le débit de la pompe d'injection 44 de ce liquide sera choisi de façon que le rapport de dilution soit d'environ 30.

Dans la description des phases de la procédure de désinfection qui suit, les pompes qui ne sont pas mentionnées ne fonctionnent pas et agissent comme des organes d'occlusion. Sauf mention contraire, la vanne 18 disposée sur la canalisation de purge 17 est fermée.

### Première phase :

La vanne 18 est ouverte et les pompes 6a, 6b d'injection du générateur de liquide de dialyse fonctionnent. La durée de cette phase est calculée pour que, compte tenu du débit des pompes 6a, 6b et 13, le générateur de liquide de dialyse, la première chambre 9 du premier filtre 8, la canalisation de recirculation 12 et la canalisation de purge 17 soient remplis totalement de liquide bactéricide à la fin de la première phase. Les pompes 6a et 6b sont alors stoppées et la vanne 18 est fermée.

### Deuxième phase :

La deuxième pompe de circulation 20 fonctionne. La vanne trois voies 34 est disposée de façon à ne pas obturer la canalisation d'évacuation 2. La durée de cette phase est calculée pour que, compte tenu du débit de la pompe 20, le deuxième filtre 21 et les canalisations 25, 26 qui lui sont connectées, la canalisation de bouclage 27, la canalisation d'évacuation 2, la canalisation de jonction 3, et la portion de canalisation d'alimentation 1 comprise entre la canalisation de bouclage 27 et la canalisation de jonction 3, soient totalement remplis de liquide bactéricide à la fin de la deuxième phase. La pompe de circulation 20 est alors arrêtée.

### Troisième phase :

La première pompe de circulation 19 fonctionne et les vannes trois voies 33, 34 sont disposées de façon à autoriser la circulation du liquide dans la canalisation d'étalonnage 32. La durée de cette phase est calculée pour que, compte tenu du débit de la pompe 19, la canalisation d'étalonnage 32 soit totalement remplie de liquide bactéricide à la fin de la troisième phase. La pompe de circulation 19 est alors stoppée et la vanne trois voies 34 est disposée de façon à rétablir le passage dans la canalisation d'évacuation 2.

### Quatrième phase :

La deuxième pompe de circulation 20 et la pompe d'ultrafiltration 35 fonctionnent. La vanne 37 du dispositif de mesure de débit d'ultrafiltration est ouverte jusqu'à ce que la canalisation de dérivation 36 soit remplie de liquide bactéricide. La vanne 37 est alors fermée de façon que la burette 38 se remplisse. La durée de cette phase est calculée pour que, compte tenu du débit de la pompe d'ultrafiltration 35, la canalisation de dérivation 36 et la burette 38 soient totalement remplies -de liquide bactéricide à la fin de cette quatrième phase.

Quand le circuit hydraulique de la machine de dialyse est totalement rempli de liquide bactéricide comme cela vient d'être décrit, toutes les pompes sont stoppées pendant une période de temps déterminée courant à partir de la fin de la première phase. Pendant cette période qui, avec l'exemple de désinfectant pris plus haut est préférablement de l'ordre de 30 minutes, le premier liquide stagne dans le circuit et exerce son action bactéricide. Cette durée est cependant beaucoup trop courte pour que le circuit ne subisse de corrosion.

Conformément à l'invention, le volume initial de liquide de désinfection concentré introduit dans le réservoir 40 est choisi pour qu'à la fin du remplissage du circuit hydraulique, il reste dans le réservoir 40 un volume résiduel déterminé de liquide de désinfection concentré.

Pendant la période de stagnation, le raccord 46 de la canalisation d'injection 43 de liquide de désinfection est ouvert et la portion de canalisation solidaire du réservoir 40 est reliée à une source d'eau. Le réservoir 40 est rempli au moyen de la pompe 44 jusqu'au niveau du détecteur 41 de niveau haut, puis la canalisation 43 est reconnectée à la canalisation d'alimentation 1. Le réservoir 40 contient alors un second liquide de désinfection dilué.

Conformément à l'invention, à la fin de la période de stagnation le circuit hydraulique est vidangé complètement du premier liquide de désinfection et il est rempli à nouveau, selon le programme à quatre phases décrit plus haut, au moyen d'un troisième liquide (ou liquide bactériostatique) résultant d'un mélange dosé d'eau et du deuxième liquide de désinfection préparé dans le réservoir 40 pendant la période de stagnation. Le rapport de dilution est choisi de telle façon que le troisième liquide soit bactériostatique et n'ait pas d'action corrosive sur le circuit même après une stagnation de plusieurs heures, au moins douze heures de préférence. Pour reprendre l'exemple de l'eau de Javel à 48° chlorométrique donné plus haut, on obtient le résultat désiré en choisissant, pour le deuxième liquide préparé dans le réservoir 40 pendant la période de stagnation, un rapport de dilution eau/liquide de désinfection concentré de environ 3,5 et en ajustant la pompe d'injection 44 de liquide de désinfection de façon que, lors du remplissage du circuit hydraulique avec le liquide bactériostatique, le rapport de dilution eau/deuxième liquide soit de environ 30.

Selon une variante de l'invention, le liquide de désinfection concentré est préparé à partir d'un volume d'eau déterminé dans lequel on fait dissoudre une quantité déterminée de désinfectant soluble sous forme de poudre, de granulés ou, de préférence, de tablettes effervescentes. Le réservoir 40 comprend alors un orifice pour l'introduction du désinfectant soluble. La préparation du liquide de désinfection concentré et du deuxième liquide utilisés respectivement pour préparer le liquide bactéricide et le liquide bactériostatique est alors légèrement différente de ce qui a été décrit plus haut en ce que le réservoir 40, dans les deux cas, est rempli au moyen d'eau mise en circulation dans la canalisation d'alimentation 1.

Dans la description qui précède, le réservoir 40 muni des détecteurs de niveau haut et bas 41 et 42 a été présenté comme une partie intégrante de la machine. Il est naturellement possible d'utiliser un conteneur extérieur à la machine qui serait fourni à l'utilisateur avec un volume déterminé de liquide de désinfection concentré correspondant au niveau haut du réservoir 40.

L'invention n'est pas limitée aux modes de réalisation spécifiques qui viennent d'être décrits et elle est susceptible de variantes, sans sortir du cadre de l'invention telle que définie par les revendications.

## Revendications

1. Procédé de désinfection du circuit hydraulique d'une machine de dialyse comprenant une entrée (E) pour raccordement à une source d'eau extérieure et une pluralité de canalisations (1, 2, 4a, 4b, 26) ouvertes prévues pour être reliées, dans une configuration opérationnelle de la machine, à différents accessoires consommables, comprenant les étapes de :
- fermer les canalisations ouvertes (1, 2, 4a, 4b, 26) sur le circuit hydraulique de façon à former un réseau clos de canalisations ;
- mettre en communication avec le circuit hydraulique, à proximité de l'entrée (E), un réservoir (40) contenant un liquide de désinfection concentré ;
- provoquer un écoulement proportionnel d'eau et de liquide de désinfection concentré dans le circuit hydraulique de façon à produire un premier liquide ayant un pouvoir bactéricide ;
- interrompre l'écoulement d'eau et de liquide de désinfection concentré lorsque le circuit hydraulique est rempli de liquide bactéricide, le volume initial de liquide de désinfection concentré dans le réservoir (40) étant choisi pour qu'à la fin du remplissage du circuit hydraulique il reste dans le réservoir (40) un volume résiduel;
- ajouter un volume d'eau au volume résiduel dans le réservoir (40) pour produire un deuxième liquide ;
- vidanger le circuit hydraulique du liquide bactéricide ;
- provoquer simultanément un écoulement proportionnel d'eau et du deuxième liquide dans le circuit hydraulique de façon à produire un troisième liquide ayant un pouvoir bactériostatique et pouvant rester dans le circuit hydraulique pendant au moins douze heures sans y causer de corrosion ;
- remplir le circuit hydraulique de liquide bactériostatique.

2. Procédé selon la revendication 1, **caractérisé en ce que**, préalablement à l'étape de mettre en communication le réservoir (40) avec le circuit hydraulique il comprend l'étape de remplir le réservoir (40) avec un volume déterminé de liquide de désinfection concentré.

3. Procédé selon la revendication 2, **caractérisé en ce que** le réservoir (40) est rempli jusqu'à un niveau haut prédéterminé (41) avec le liquide de désinfection concentré.

4. Procédé selon la revendication 1, **caractérisé en ce que**, préalablement à l'étape de mettre en communication le réservoir (40) avec le circuit hydraulique il comprend l'étape de remplir le réservoir (40) avec un volume déterminé d'eau et d'y introduire une quantité déterminée de désinfectant soluble sous forme de poudre, de granulés ou de tablettes.

5. Procédé selon une des revendications 1 à 4, **caractérisé en ce que** l'étape de remplissage du circuit hydraulique avec le liquide bactéricide et l'étape de remplissage du circuit hydraulique avec le liquide bactériostatique sont effectuées en plusieurs phases correspondant au remplissage de plusieurs portions du circuit hydraulique.

6. Procédé selon la revendication 5, pour un circuit hydraulique comprenant un générateur de liquide de dialyse (4a, 5a, 6a, 7a ; 4b, 5b, 6b, 7b) **caractérisé en ce que** une première phase de remplissage comprend le remplissage du générateur de liquide de dialyse (4a, 5a, 6a, 7a ; 4b, 5b, 6b, 7b).

7. Procédé selon une des revendications 4 et 5, pour un circuit hydraulique comprenant un premier filtre (8) destiné à la filtration d'un liquide de dialyse, **caractérisé en ce que** une deuxième phase de remplissage comprend le remplissage du premier filtre (8).

8. Procédé selon une des revendications 4 à 7, pour un circuit hydraulique comprenant un deuxième filtre (21) destiné à la filtration d'un liquide de perfusion, **caractérisé en ce que** une troisième phase de remplissage comprend le remplissage du deuxième filtre (21).

9. Procédé selon une des revendications 4 à 8, pour un circuit hydraulique comprenant un dispositif d'extraction (35, 36, 37, 38) d'un liquide hors d'une section du circuit destinée à être connectée, dans une configuration opérationnelle de la machine, à un dialyseur, **caractérisé en ce que** une quatrième phase de remplissage comprend le remplissage du dispositif d'extraction (35, 36, 37, 38).

10. Procédé selon une des revendications 1 à 9, **caractérisé en ce que** la vidange du liquide bactéricide est provoquée par le remplissage du circuit hydraulique avec le liquide bactériostatique.

11. Procédé selon une des revendications 1 à 10, **caractérisé en ce que** l'étape de remplissage du circuit hydraulique avec le liquide bactériostatique est précédé d'une étape de stagnation du liquide bactéricide dans le circuit.

## Patentansprüche

1. Verfahren zur Desinfektion des Hydraulikkreises einer Dialysemaschine mit einem Einlaß (E) zur Verbindung mit einer äußeren Wasserquelle und mehreren offenen Leitungen (1, 2, 4a, 4b, 26), die in einer Betriebskonfiguration der Maschine mit verschiedenem Verbrauchsstoffzubehör verbunden werden sollen, bei dem man
- die offenen Leitungen (1, 2, 4a, 4b, 26) so am Hydraulikkreis schließt, daß sie ein geschlossenes Leitungsnetz bilden;
- einen eine konzentrierte Desinfektionsflüssigkeit enthaltenden Behälter (40) in der Nähe des Einlasses (E) mit dem Hydraulikkreis in Verbindung setzt;
- einen proportionalen Strom aus Wasser und konzentrierter Desinfektionsflüssigkeit im Hydraulikkreis bewirkt, so daß eine erste Flüssigkeit mit einer bakteriziden Wirkung erzeugt wird;
- den Strom aus Wasser und konzentrierter Desinfektionsflüssigkeit unterbricht, wenn der Hydraulikkreis mit bakterizider Flüssigkeit gefüllt ist, wobei das Anfangsvolumen der konzentrierten Desinfektionsflüssigkeit im Behälter (40) so gewählt wird, daß am Ende des Füllens des Hydraulikkreises ein Restvolumen im Behälter (40) verbleibt;
- ein Wasservolumen zum Restvolumen im Behälter (40) hinzufügt, um eine zweite Flüssigkeit zu erzeugen;
- die bakterizide Flüssigkeit aus dem Hydraulikkreis abläßt;
- gleichzeitig im Hydraulikkreis einen proportionalen Strom aus Wasser und der zweiten Flüssigkeit bewirkt, so daß eine dritte Flüssigkeit mit einer bakteriostatischen Wirkung erzeugt wird, die mindestens zwölf Stunden lang im Hydraulikkreis verbleiben kann, ohne dort Korrosion zu verursachen;
- den Hydraulikkreis mit bakteriostatischer Flüssigkeit füllt.

2. Verfahren nach Anspruch 1, **dadurch gekennzeichnet, daß** man vor dem Inverbindungsetzen des Behälters (40) mit dem Hydraulikkreis den Behälter (40) mit einem bestimmten Volumen einer konzentrierten Desinfektionsflüssigkeit füllt.

3. Verfahren nach Anspruch 2, **dadurch gekennzeichnet, daß** man den Behälter (40) auf ein vorbestimmtes oberes Niveau (41) mit der konzentrierten Desinfektionsflüssigkeit füllt.

4. Verfahren nach Anspruch 1, **dadurch gekennzeichnet, daß** man vor dem Inverbindungsetzen des Behälters (40) mit dem Hydraulikkreis den Behälter (40) mit einem bestimmten Wasservolumen füllt und eine bestimmte Menge an lösbarem Desinfektionsmittel in Form von Pulver, von Körnchen oder Tabletten darin einleitet.

5. Verfahren nach einem der Ansprüche 1 bis 4, **dadurch gekennzeichnet, daß** das Füllen des Hydraulikkreises mit der bakteriziden Flüssigkeit und das Füllen des Hydraulikkreises mit der bakteriostatischen Flüssigkeit in mehreren Phasen, die dem Füllen mehrerer Teile des Hydraulikkreises entsprechen, durchgeführt wird.

6. Verfahren nach Anspruch 5 für einen Hydraulikkreis, der einen Dialyseflüssigkeitserzeuger (4a, 5a, 6a, 7a; 4b, 5b, 6b, 7b) umfaßt, **dadurch gekennzeichnet, daß** in einer ersten Füllphase der Dialyseflüssigkeitserzeuger (4a, 5a, 6a, 7a; 4b, 5b, 6b, 7b) gefüllt wird.

7. Verfahren nach Anspruch 4 oder 5 für einen Hydraulikkreis, der ein erstes Filter (8) umfaßt, das eine Dialyseflüssigkeit filtern soll, **dadurch gekennzeichnet, daß** bei einer zweiten Füllphase das erste Filter (8) gefüllt wird.

8. Verfahren nach einem der Ansprüche 4 bis 7 für einen Hydraulikkreis, der ein zweites Filter (21) umfaßt, das eine Perfusionsflüssigkeit filtern soll, **dadurch gekennzeichnet, daß** bei einer dritten Füllphase das zweite Filter (21) gefüllt wird.

9. Verfahren nach einem der Ansprüche 4 bis 8 für einen Hydraulikkreis, der eine Extraktionsvorrichtung (35, 36, 37, 38) zum Extrahieren einer Flüssigkeit aus einem Abschnitt des Kreises umfaßt, der in einer Betriebskonfiguration der Maschine mit einem Dialysator verbunden werden soll, **dadurch gekennzeichnet, daß** bei einer vierten Füllphase die Extraktionsvorrichtung (35, 36, 37, 38) gefüllt wird.

10. Verfahren nach einem der Ansprüche 1 bis 9, **dadurch gekennzeichnet, daß** das Ablassen der bakteriziden Flüssigkeit durch das Füllen des Hydraulikkreises mit der bakteriostatischen Flüssigkeit bewirkt wird.

11. Verfahren nach einem der Ansprüche 1 bis 10, **dadurch gekennzeichnet, daß** dem Füllen des Hydraulikkreises mit der bakteriostatischen Flüssigkeit ein Stagnieren der bakteriziden Flüssigkeit im Kreis vorausgeht.

## Claims

1. Method for disinfection of the hydraulic circuit of a dialysis machine, comprising an inlet (E) for connection to an external water source and a plurality of open lines (1, 2, 4a, 4b, 26) intended to be connected, in an operational configuration of the machine, to different consumable accessories, comprising the steps of:
- closing the open lines (1, 2, 4a, 4b, 26) on the hydraulic circuit in such a way as to form a closed network of lines;
- bringing a reservoir (40) containing a concentrated disinfection liquid into communication with the hydraulic circuit, in proximity to the inlet (E);
- instigating a proportional flow of water and of concentrated disinfection liquid in the hydraulic circuit in such a way as to produce a first liquid having a bactericidal action;
**characterized in that** it comprises the steps of:
- interrupting the flow of water and of concentrated disinfection liquid when the hydraulic circuit is filled with bactericidal liquid, the initial volume of concentrated disinfection liquid in the reservoir (40) being chosen such that at the end of the filling of the hydraulic circuit, a residual volume remains in the reservoir (40);
- adding a volume of water to the residual volume in the reservoir (40) in order to produce a second liquid;
- emptying the hydraulic circuit of the bactericidal liquid;
- instigating simultaneously a proportional flow of water and of the second liquid in the hydraulic circuit in such a way as to produce a third liquid having a bacteriostatic action and capable of remaining in the hydraulic circuit for at least twelve hours without causing corrosion there;
- filling the hydraulic circuit with bacteriostatic liquid.

2. Method according to Claim 1, **characterized in that** prior to the step of bringing the reservoir (40) into communication with the hydraulic circuit, it comprises the step of filling the reservoir (40) with a defined volume of concentrated disinfection liquid.

3. Method according to Claim 2, **characterized in that** the reservoir (40) is filled to a predetermined upper level (41) with the concentrated disinfection liquid.

4. Method according to Claim 1, **characterized in that** prior to the step of bringing the reservoir (40) into communication with the hydraulic circuit, it comprises the step of filling the reservoir (40) with a defined volume of water and of introducing thereto a defined quantity of soluble disinfectant in the form of a powder, granules or tablets.

5. Method according to one of Claims 1 to 4, **characterized in that** the step of filling the hydraulic circuit with the bactericidal liquid and the step of filling the hydraulic circuit with the bacteriostatic liquid are performed in several phases corresponding to the filling of several portions of the hydraulic circuit.

6. Method according to Claim 5, for a hydraulic circuit comprising a dialysis liquid generator (4a, 5a, 6a, 7a; 4b, 5b, 6b, 7b), **characterized in that** a first filling phase comprises the filling of the dialysis liquid generator (4a, 5a, 6a, 7a; 4b, 5b, 6b, 7b).

7. Method according to one of Claims 4 and 5, for a hydraulic circuit comprising a first filter (8) intended for the filtration of a dialysis liquid, **characterized in that** a second filling phase comprises the filling of the first filter (8).

8. Method according to one of Claims 4 to 7, for a hydraulic circuit comprising a second filter (21) intended for the filtration of a perfusion liquid, **characterized in that** a third filling phase comprises the filling of the second filter (21).

9. Method according to one of Claims 4 to 8, for a hydraulic circuit comprising an extraction device (35, 36, 37, 38) for extracting a liquid from a section of the circuit intended to be connected, in an operational configuration of the machine, to a dialyser, **characterized in that** a fourth filling phase comprises the filling of the extraction device (35, 36, 37, 38).

10. Method according to one of Claims 1 to 9, **characterized in that** the emptying of the bactericidal liquid is instigated by the filling of the hydraulic circuit with the bacteriostatic liquid.

11. Method according to one of Claims 1 to 10, **characterized in that** the step of filling the hydraulic circuit with the bacteriostatic liquid is preceded by a step involving stagnation of the bactericidal liquid in the circuit.
